# EUROPÄISCHE PATENTSCHRIFT

(11) **EP 2 817 063 B1**
(45) Veröffentlichungstag und Bekanntmachung des Hinweises auf die Patenterteilung: **23.12.2015**
(21) Anmeldenummer: 13707535.4
(22) Anmeldetag: 14.01.2013
(51) Int. Cl.: A61N 1/05

(54) **MYOKARDIALE HERZSCHRITTMACHER ELEKTRODE**
MYOCARDIAL HEART PACEMAKER ELECTRODE
ÉLECTRODE MYOCARDIQUE POUR STIMULATEUR CARDIAQUE

(30) Priorität: 24.02.2012 DE 202012001945 U
(43) Veröffentlichungstag der Anmeldung: 31.12.2014
(73) Patentinhaber: Osypka, Peter, 79639 Grenzach-Wyhlen (DE)
(72) Erfinder: Osypka, Peter, 79639 Grenzach-Wyhlen (DE)
(86) Internationale Anmeldenummer: PCT/DE2013/000016
(87) Internationale Veröffentlichungsnummer: WO 2013/123924

(56) Entgegenhaltungen:
- EP-A1- 0 083 674
- EP-A1- 0 668 087
- EP-A2- 0 234 457
- WO-A1-2005/039691
- US-A- 5 716 392
- US-B1- 7 920 928

## Beschreibung

Die Erfindung betrifft eine bipolare myokardiale permanente Herzschrittmacher Elektrode, besonders für Kinder geeignet, mit einer minimalen Fixierung und einem in der Gebrauchslage an der Außenseite des Herzens im Herzgewebe (Myokard) tangential angeordneten Pol mit einer zu einem Herzschrittmacher verlaufenden Zuleitung.

Myokardiale Herzschrittmacher Elektroden sind aus EP 0 083 674, DE 2 219 044.9 aus US 4 355 642 und aus WO 2005/039691 bereits bekannt.
EP0 083 674 beschreibt eine temporäre myokardiale Herzschrittmacher Elektrode bestehend aus Stecker, Zuleitung, chirurgischem Faden mit Fixierungselement in Form einer Wendel. Über die Wendel ist der Faden mit der Elektrode fest verankert. Der Faden dient dazu die Helix auseinander zu ziehen.
WO2005039691 beschreibt eine myokardiale Elektrode mit einem speziellen Befestigungsmechanismus in Form eines Klappmechanismus.

Für Kinder mit einem angeborenen Herzfehler, die nach der Operation einen Herzschrittmacher benötigen, sind die bisherigen Elektroden im Vergleich zur Herzgröße unverhältnismäßig groß und können zu großen Problemen führen. Für die bipolare Stimulation müssen oft sogar zwei Exemplare von den derartig großen Elektroden auf dem Herzen des Babys fixiert werden.

Es besteht daher die Aufgabe, eine myokardiale Elektrode der eingangs genannten Art zu schaffen, bei welcher die Größe und die Fixierung der Elektrode einem Kinderherzen angepasst ist.

Zur Lösung dieser Aufgabe wird eine bipolare myokardiale Herzschrittmacher Elektrode (1) vorgeschlagen, bestehend aus Stecker (2), Zuleitung (3), Kathode (4), Anode (5), chirurgischem Faden (7) und einem Fixierungselement (8). Die Elektrode ist dadurch gekennzeichnet, dass die Elektrode (1) ein inneres Lumen aufweist und auf dem Faden (7) frei beweglich ist;
der Faden (7) durch das innere Lumen der Elektrode einschließlich ihrer Zuleitung und Stecker durchführbar ist und distal das Fixierungselement (8) in Form eines Knotens trägt,
die Zuleitung (3) außen isoliert ist und distal einen in der Gebrauchslage an der Außenseite des Herzens im Herzgewebe (Myokard) tangential angeordneten Kathoden-Pol (4) aufweist, wobei der Kathoden-Pol aus einer konisch zulaufenden Wendel besteht und die Zuleitung (3) proximal einen in der Gebrauchslage unterhalb des Herzens angeordneten Anodenpol (5) aufweist.

Der chirurgische Faden wird mittels einer Herznadel an der geeigneten Stelle im Myokard eingeführt. An der Stelle, an der die Nadel aus dem Myokard herausgezogen wird, wird ein Knoten gemacht und der Rest von Faden und Nadel entfernt. Der Knoten dient als Fixierungselement (Widerhaken). Der Faden ist von Hersteller-Seite bereits durch das innere Lumen der Elektrode einschließlich ihrer Zuleitung und Stecker durchgeführt, sodass die Elektrode auf dem Faden frei beweglich ist.

Der als dünne Wendel ausgeführte Pol (Kathode (4)) der Elektrode kann jetzt über den Faden, der als Führung dient, leicht in das Myokard hinein gezogen werden. Der aus dem Elektroden Stecker (2) herausragende Restfaden wird im Stecker Stift verankert. Die Anode (5) (indifferenter Pol) der bipolaren myokardialen Elektrode ist auf der Elektroden Zuleitung proximal als Wendel unterhalb des Herzens angeordnet.

### Vorteile:

Die myokardiale Herzschrittmacher Elektrode wird allein durch einen kleinen Knoten eines chirurgischen Fadens fixiert und die Anode wird unterhalb des Herzens platziert. Eine zweite separate Elektrode als Anode ist überflüssig. Dadurch dass die Elektrode an dem Faden leicht beweglich ist kann die Kathode (4) optimal platziert werden.

### Zeichnungen

Weitere Einzelheiten, Merkmale und Vorteile der Erfindung lassen sich im folgenden Beschreibungsteil entnehmen, in dem anhand der Zeichnungen und Ausführungsbeispiele die Erfindung näher erläutert wird.
Sie zeigen in schematischer Darstellung in
**Fig. 1** eine beispielhafte Ausführung der myokardialen Herzschrittmacher Elektrode in der ein chirurgischen Faden eingeführt ist.
**Fig. 2** eine beispielhafte Ausführung der myokardialen Herzschrittmacher Elektrode bei der durch das Myokard ein chirurgischer Faden eingeführt ist.
**Fig. 3** eine beispielhafte Ausführung der myokardialen Herzschrittmacher Elektrode bei der mit Hilfe des chirurgischen Fadens ein Knoten gemacht worden ist, der Restfaden entfernt und der Knoten bis an die äußere Wand des Herzens zurückgezogen ist.
**Fig. 4** eine beispielhafte Ausführung der myokardialen Herzschrittmacher Elektrode bei der über den chirurgischen Faden der distale Pol (Kathode) der Elektrode in das Myokard eingezogen ist.
**Fig. 5** eine beispielhafte Ausführung der myokardialen Herzschrittmacher Elektrode bei der der proximale Teil des chirurgischen Fadens im Stecker Stift verankert ist.

### Beschreibung der Zeichnungen:

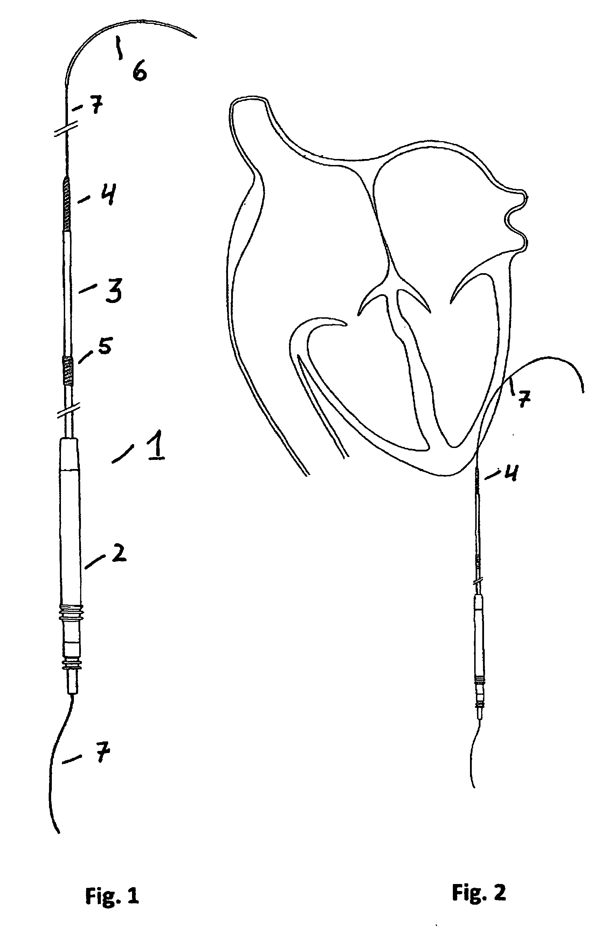

**Fig.1** zeigt eine beispielhafte Ausführung der myokardialen Herzschrittmacher Elektrode (1), bestehend aus Stecker (2), Schaft oder Zuleitung (3), Kathode (4), Anode (5), Herznadel (6) und dem chirurgischen Faden (7), der durch die gesamte Länge der Elektrode eingeführt ist.

Das Material der Pole (Kathodenpol (4), Anodenpol (5)) besteht aus einem biokompatiblen elektrisch leitfähigen Material. Beispielhaft sei genannt Platin, Edelstahl oder MP 35N.

Die Zuleitung (3) der Elektrode (1) besteht aus einer Wendel mit mindestens zwei parallel gewickelten isolierten Drähten oder aus mindestens einer Wendel und einer isolierten Litze. Die Zuleitung (3) der Elektrode (1) kann auch aus mindestens zwei isolierten Litzen bestehen.

Die Zuleitung (3) muss nach außen hin isoliert sein. Die äußere Isolation der Zuleitung besteht beispielsweise aus einem Schaft Schlauch aus Silikon oder Polyurethan.

Es ist vorteilhaft wenn der Rest des Fadens (7) fixiert ist. Dazu trägt der Stecker Pin (10) einen Schlitz, in dem der Rest des chirurgischen Fadens fixierbar ist.

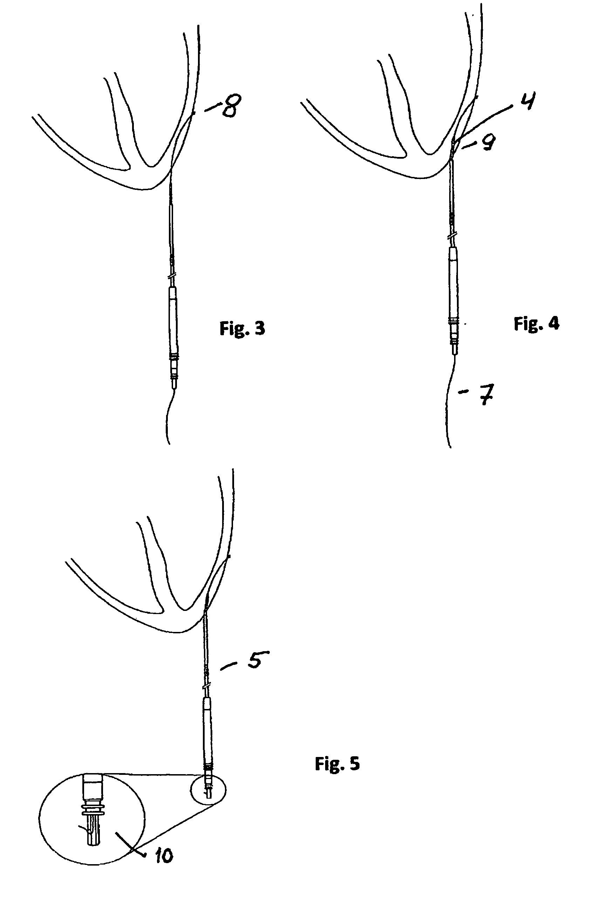

**Fig. 2** zeigt eine beispielhafte Ausführung der myokardialen Herzschrittmacher Elektrode (1) bei der durch das Myokard ein chirurgischer Faden (7) eingeführt ist. Die Kathode (4) wird nach der in Fig. 3 gezeigten Fixierung mit Hilfe des Fadens (7) in das Myokard gezogen und platziert.

**Fig. 3** zeigt eine beispielhafte Ausführung der myokardialen Herzschrittmacher Elektrode (1) bei der mit Hilfe des chirurgischen Fadens (7) ein Knoten (8) gemacht, der Restfaden entfernt und der Knoten bis an die äußere Wand des Herzens zurückgezogen worden ist.

**Fig. 4** zeigt eine beispielhafte Ausführung der myokardialen Herzschrittmacher Elektrode (1) bei der über den chirurgischen Faden (7) der distale Pol (Kathode) (4) der Elektrode in das Myokard (9) eingezogen ist.

**Fig. 5** zeigt eine beispielhafte Ausführung der myokardialen Herzschrittmacher Elektrode (1) bei der der proximale Teil des chirurgischen Fadens im Stecker Stift (10) verankert ist. Die Anode (5) der Elektrode ist als Wendel ausgebildet und liegt in Gebrauchslage unterhalb des Herzens.

## Patentansprüche

1. Bipolare myokardiale Herzschrittmacher Elektrode (1), bestehend aus Stecker (2), Zuleitung (3), Kathode (4), Anode (5), chirurgischem Faden (7) und einem Fixierungselement (8), wobei die Zuleitung (3) außen isoliert ist und distal einen in der Gebrauchslage an der Außenseite des Herzens im Herzgewebe (Myokard) tangential angeordneten Kathoden-Pol (4) aufweist, wobei der Kathoden-Pol aus einer konisch zulaufenden Wendel besteht und die Zuleitung (3) proximal einen in der Gebrauchslage unterhalb des Herzens angeordneten Anodenpol (5) aufweist
**dadurch gekennzeichnet, dass** die Elektrode (1) ein inneres Lumen aufweist und auf dem Faden (7) frei beweglich ist;
der Faden (7) durch das innere Lumen der Elektrode einschließlich ihrer Zuleitung, die aus einer Wendel mit mindestens zwei parallel gewickelten isolierten Drähten besteht,
und durch den Stecker durchführbar ist und distal das Fixierungselement (8) in Form eines Knotens trägt.

2. Bipolare myokardiale Herzschrittmacher Elektrode nach Anspruch 1, **dadurch gekennzeichnet, dass** das Material der Pole aus einem biokompatiblen elektrisch leitfähigen Material besteht, wie aus Platin, Edelstahl, oder MP 35N.

3. Bipolare myokardiale Herzschrittmacher Elektrode nach Anspruch 1, **dadurch gekennzeichnet, dass** die äußere Isolation der Zuleitung aus einem Schaft Schlauch aus Silikon oder Polyurethan besteht.

4. Bipolare myokardiale Herzschrittmacher Elektrode nach Anspruch 1 bis 3, **dadurch gekennzeichnet, dass** der Stecker Pin einen Schlitz enthält, in dem der Rest des chirurgischen Fadens fixierbar ist.

## Claims

1. Bipolar myocardial heart pacemaker electrode (1), consisting of a plug (2), a supply line (3), a cathode (4), an anode (5), a surgical thread (7) and a fixation element (8), whereby the supply line (3) is externally insulated and has a distal cathode pole (4) tangentially arranged on the outside of the heart in the heart tissue (myocardium) in the usage position, whereby the cathode pole consists of a conically tapering coil and the supply line (3) has an anode pole (5) arranged proximally below the heart in the usage position,
**characterized in that** the electrode (1) has an internal lumen and is freely movable on the thread (7); the thread (7) can be guided through the inner lumen of the electrode including the supply line and plug thereof, whereby the supply line consists of a helix with at least two parallel wound insulated wires and whereby the thread comprises a distally arranged fixation element (8) in form of a knot.

2. Bipolar myocardial heart pacemaker electrode according to claim 1 **characterized in that** the material of the pole consists of a biocompatible electrically conductive material, such as platinum, stainless steel, or MP 35N.

3. Bipolar myocardial pacemaker electrode according to claim 1, **characterized in that** the outer insulation of the supply line consists of a shaft tube made of silicone or polyurethane.

4. Bipolar myocardial pacemaker electrode according to claim 1 to 3, **characterized in that** the plug has a slot in which the rest of the surgical thread can be fixed.

## Revendications

1. Électrode myocardique bipolaire (1) pour stimulateur cardiaque constituée d'une prise mâle (2), d'une conduite d'alimentation (3), d'une cathode (4), d'une anode (5), d'un fil chirurgical (7) et d'un élément de fixation (8),
la conduite d'alimentation (3) est isolée de l'extérieur et comporte de manière distale un pôle de cathode (4) disposé dans la position d'utilisation de manière tangentielle sur la face extérieure du coeur dans le tissu cardiaque (myocarde); le pôle de cathode est constitué d'un filament se terminant de manière conique et la conduite d'alimentation (3) comporte de manière proximale un pôle d'anode (5) disposé, dans la position d'utilisation, au-dessous du coeur,
l'électrode (1) est **caractérisée en ce qu'**elle comporte une lumière intérieure et, d'autre part, une position sur le fil (7) ajustable ; le fil (7) peut être guidé à travers la lumière intérieure de l'électrode, ainsi qu'à travers de sa conduite d'alimentation et sa prise mâle, et porte l'élément de fixation (8) de manière distale sous forme de noeud.

2. Électrode myocardique bipolaire pour stimulateur cardiaque selon la revendication 1, **caractérisée en ce que** les pôles sont constitués d'un matériau biocompatible et électroconducteur, tel que le platine, l'acier inoxydable ou MP 35N.

3. Électrode myocardique bipolair pour stimulateur cardiaque selon la revendication 1, **caractérisée en ce que** l'isolation extérieure du conducteur se compose d'un tube en silicone ou en polyuréthane.

4. Électrode myocardique bipolaire pour stimulateur cardiaque selon la revendication 1 à 3, **caractérisée en ce que** le connecteur comprend une fente dans laquelle le reste du fil chirurgical peut être fixé.
